# EUROPEAN PATENT SPECIFICATION

(11) **EP 4 320 430 B1**
(45) Date of publication and mention of the grant of the patent: **04.03.2026**
(21) Application number: 22716366.4
(22) Date of filing: 17.03.2022
(51) Int. Cl.: G01N 27/12, G01N 33/00

(54) **HALOGENATED GAS SENSOR**
SENSOR FÜR HALOGENHALTIGE GASE
CAPTEUR DE GAZ HALOGÉNÉ

(30) Priority: 07.04.2021 US 202163171661 P
(43) Date of publication of application: 14.02.2024
(73) Proprietor: Inficon GmbH, 50968 Köln (DE)
(72) Inventor: GU, Gang, Syracuse, New York 13057 (US)
(74) Representative: dompatent
(86) International application number: PCT/EP2022/057018
(87) International publication number: WO 2022/214293

(56) References cited:
- US-A- 3 751 968
- US-A- 5 104 513
- US-A- 5 226 309
- US-A- 5 932 176

## Description

### Background of the Invention

Materials with at least one component with a chemical formula of NaAlSiO4 or KAISiO4 or RbAlSiO4 or CsAlSiO4 were used to fabricate bead type sensor for highly sensitive and selective sensor for the detection of halogenated gas. The sensor contains a center electrode, a coil and sensing material. The coil is heated by current passing through it. The sensing material is porous. The conductance between center electrode and heated coil changes with concentration of halogenated gas.

This invention relates to improvement of bead type sensor for highly sensitive detection of halogenated gases, especially refrigerant gases such as hydrofluoroolefin (HFO) and hydroflurocarbons (HFCs), which show a much less global warming potential compared to hydrochlorofluorocarbons (HCFCs).

There are several existing technologies for halogenated gas detection. Tin oxide based metal oxide semiconductor (MOS) sensors have been used for halogenated gas detection; however these sensors show cross sensitivity to many hydrocarbons and humidity. Non-dispersive infrared (NDIR) optical sensors are used for halogenated gas detection as well; however these sensors show limited sensitivity and are expensive to produce.

Solid state bead type sensors to detect halogenated gases are relatively cheap to produce. These sensors are disclosed by Loh in US Pat. No. 3751968, by Lee in US Pat. No. 5104513, by Stetter in US Pat. No. 5226309 and by Yannopoulos in US Pat. No. 5932176. Loh disclosed a sensing element comprises a glass-ceramic comprising a mixture of lanthanum oxide, lanthanum fluoride, and sodium silicate. Lee disclosed a sensing element of a ceramic, comprising a mixture of potassium silicate and compound selected from the group of silicon dioxide and aluminum oxide. Stetter disclosed a sensing material comprising sodium lanthanum fluoride silicate, having the chemical formula NaLa(SiO4)3F. Yannopoulos disclosed a sensing element comprising of sodium titanate.

The operating temperature of sensor described with US Pat. No. 5226309 is from 500 °C to 600 °C, which is too low to detect HFOs and HFCs with decent sensitivity. Lee disclosed a sensing element of ceramic, comprising a mixture of potassium silicate and aluminum oxide in a ratio of between about 0.25-4.0 parts potassium silicate by weight to 1 part aluminum oxide by weight. The ratio is broad and the phase of sensing material is not well defined, hence a reproducible sensor performance is not easy to achieve. The object of this invention is to find defined materials for selective and sensitive detection of HFOs and HFCs. The other object of this invention is to find sensing materials with high melting temperature, therefore operating of the sensor with high sensitivity at high temperature from 800 C to 1000 C is possible.

The halogenated gas sensor of the invention is defined by independent claim 1.

The sensing material may be in the form of a bead, in which the two electrodes are at least partially embedded.

One of said two electrodes may be a coil surrounding the other of the two electrodes as a center electrode.

The first electrode may be made of or comprises platinum and/or where the second electrode may be made of or comprises platinum.

A voltage source may be connected to at least the first electrode to heat the electrode by current or voltage applied to said electrode to a temperature in the range of 400°C-1200°C, 400°C-1000°C, 600°C-1200°C or 600°C-1000°C.

The first sensor may be in the form of a coil having a first end and an opposing second end, said two ends being connected to a voltage source (1), said second electrode being a center electrode in the form of a longitudinal straight element or bar extending through the center of the coil (4) along the longitudinal axis of the coil, said coil and center electrode being surrounded by and embedded in said sensing material.

The invention also provides for a method of detecting halogenated gas with a sensor as described above, wherein said sensing material is heated to a temperature in the range of 400°C-1000°C, 400°-1200°C, 600°C-1000°C or 600°C-1200°C by applying a current or voltage to said first sensor or coil.

The current through the coil after exposing the sensor to the gas to be detected may be divided by the current through the coil before the sensor is exposed to the gas to be detected.

Moreover, the invention provides for a method of manufacturing a halogenated gas sensor as described above, wherein the sensing material comprises at least a first component (A) made from a molecular sieve (3A), which is heated to a first temperature of several hundred °C, maintained at said first temperature for several, and preferably 3 hours, thereafter being heated to a second temperature, which is higher than the first temperature and kept at said second temperature for a second time, which preferably corresponds to said first time, characterized in that said first component contains NaAlSiO₄ and KAlSiO₄.

Said first component may be subsequently ground to fine particles with an average size of below 5 µm, preferably about 3 µm.

Said first component may contain NaAlSiO4 and KAISiO4 at a ratio of 1:1.

The sensing material may comprise at least a second component (B) which is prepared with an ion exchange performed with a molecular sieve (4A) and CsNO₃, said molecular sieve and CsNO₃ preferably being mixed in deionized water.

The mixed suspension of molecular sieve, CsNO₃ and deionized water may be stirred for several, and preferably 24, hours, whereafter the suspension is preferably centrifuged, thereafter preferably being heated to a first temperature of several hundred °C and preferably about 900°C for at least one hour and preferably for two hours, and thereafter heated to a second temperature higher than said first temperature, preferably to 1100°C, for several additional hours, and preferably for about 3 hours.

Said second component (B) may be ground after said heat treatment to fine particles with an average size of a few and preferably about 4 µm.

Said first component (A) and/or said second component (B) may be mixed with a vehicle to a slurry, said vehicle preferably being in the range of 5%-10% weight hydroxypropyl cellulose dissolved in water, the weight ratio of the mixture of said components (A) and/or (B) to said vehicle being about 2:1.

### Brief Description of the Drawings

In the following exemplary embodiments of the invention are described with reference to the figures, in which
- Fig.1: is a schematic view of a sensor according to an embodiment of the invention,
- Fig. 2: shows the steps of a typical process to fabricate the sensor with exemplic sensing materials,
- Fig. 3: shows scanning electron microscopy images with 30 and 1600 magnifications,
- Fig. 4: shows a typical sensor response to 100 ppm R134a and 100 ppm R1234yf,
- Fig. 5: shows a typical sensor response to different concentration of R134a, and
- Fig. 6: shows a typical sensor response to different gas/vapor.

### Detailed Description of The Invention

### Sensor Components

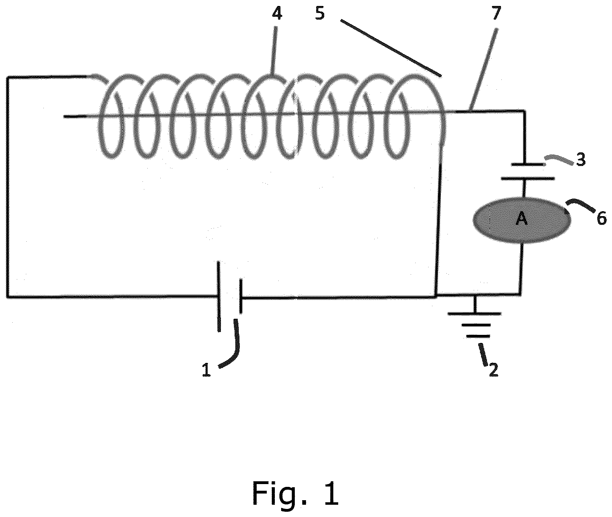

Schematic diagram of Fig. 1 shows the invented sensor comprises a center electrode 7, preferably a platinum wire, which is surrounded with a metal coil 4, preferably a platinum coil. Both are embedded in bead 5, which contains at least NaAlSiO4 and KAlSiO4.

The coil is heated by applied voltage 1 to temperature from 400 °C to 1000 °C. The current 6 is created with an applied voltage 3 between center electrode 7 and coil 4. The sensor nominal resistance is ratio of voltage 2 and current 6, which changes with halogenated gas concentration surrounding the sensor.

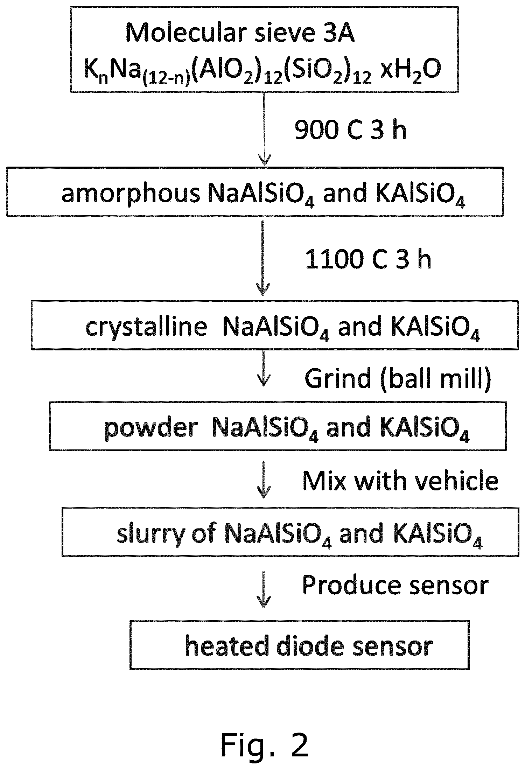

Material A for the bead 5 is synthesized with starting material of molecular sieve 3A with linear formula KnNa12-n[(AlO2)12(SiO2)12]·xH2O (n about 6) from Alfa Aesar. The process for sensor fabrication is shown in Fig. 2. For example, 50 g molecular sieve 3A was placed in a furnace and heated to 900 C with a ramping rate of 5 °C/min and kept at 900 °C for 3 hours, afterwards was heated to 1100 °C with a ramping rate of 5 °C/min and kept at the same temperature for 3 hours. Waiting for the material cool to room temperature, it was ground with a planetary ball mill (Retsch PM100) to fine particles with an average size about 3 um. XRD diffraction spectra of ground material were collected with PANalytical X'Pert PRO XRD system. It is confirmed that it only contains NaAlSiO4, KAlSiO4. Element analysis with energy-dispersive X-ray spectroscopy (EDS) confirmed that ratio of NaAlSiO4 to KAISiO4 is 1:1. Material B, not forming part of the claimed invention, was prepared with ion exchange, which was performed with molecular sieve 4A (Na12[(AlO2)12(SiO2)12] • nH2O from Alfa Aesar) and CsNO3. Typically 5 g molecular sieve 4A and 16.5 g CsNO3 mixed in 85 mL deionized water. The pH value was adjusted to 8 with NaOH solution. The suspension was stirred for 24 hours. Afterwards the suspension was centrifuged. The remaining material was ion exchanged with 16.5 g CsNO3 and 85 mL water solution for additional two times, the final remaining material was washed with deionized water for 3 times and dried at 80 °C in air overnight. Additional heat-treatment in air was performed to the material, which includes heating to 900 °C for 2 hours and additional 3 hours at 1100 °C. After the heat-treatment, the material was ground with pestle and mortar. Fine particles with average size of about 4 um are obtained. Element analysis with energy-dispersive X-ray spectroscopy (EDS) indicated that ratio of NaAlSiO4 to CsAlSiO4 is 1:1. NaAlSiO4 and KAISiO4 show melting points of 1526 °C and 1750 °C, respectively. Therefore they are expected to show long lifetime with operating temperature of up to 1000 °C.

### Sensor Fabrication

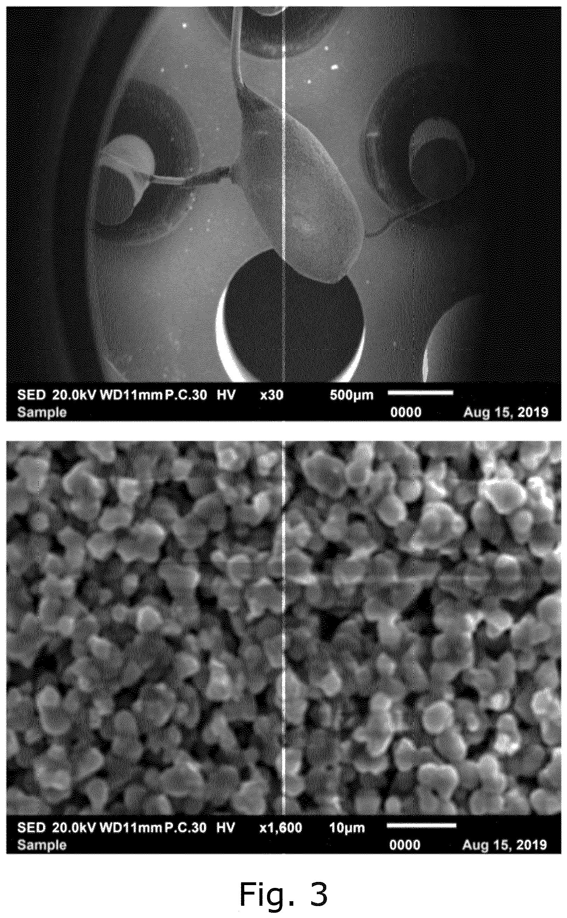

Material A or (and) Material B was (were) mixed with vehicle to get slurry. The vehicle is 5% to 10% (weight) hydroxypropyl cellulose dissolved in water. Weight ratio of Material to vehicle is about 2:1. The slurry is coated on center electrode first and wait until the coating is dry. Then the coated center electrode is inserted to heating coil. Additional slurry is added around coil to form a complete bead to cover heating coil. Afterwards the finished sensor was heated to 850 °C for 0.5-2 hours with ramping rate of 1-5 °C/min. After sensors cool to room temperature, they are ready for tests. Sensor prepared with Material A/B is denoted as Sensor A/B. Fig. 3 shows SEM images of a typical Sensor A with 30 and 1600 magnification. High magnification image shows very porous morphology.

### Sensor Performance

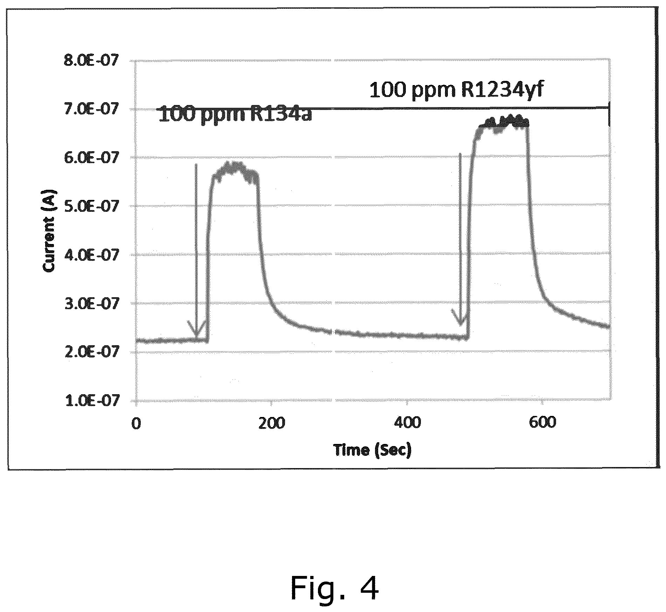

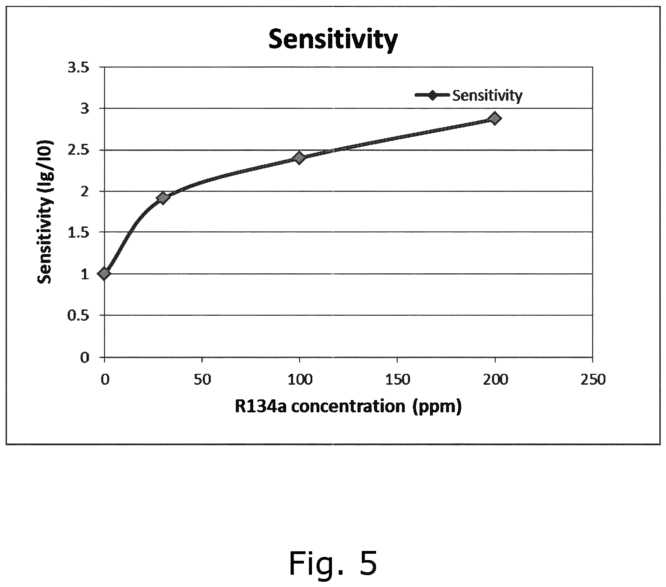

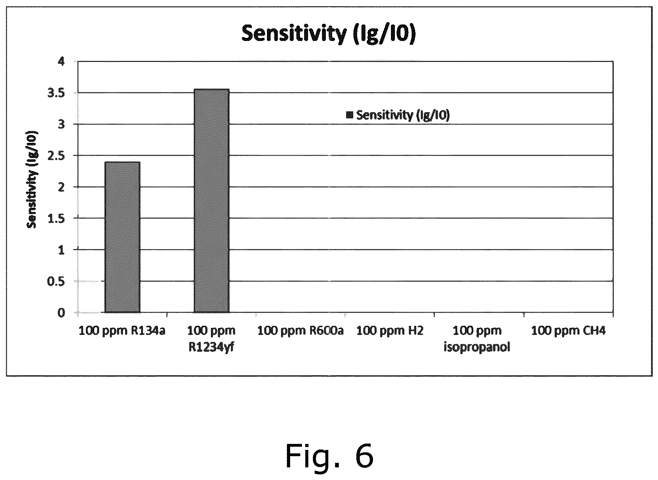

Sensor A is heated to about 800 °C by passing current through coil during operation. The current (6) in Fig. 1 changes with refrigerant gas concentration. The Current before gas exposure is denoted as I0 and the current after gas exposure is denoted as Ig. The ratio of Ig/IO is defined as sensitivity. Fig. 4 shows that Sensor A responses very quickly to 100 ppm R134a (CH2FCF3) and 100 ppm R1234yf (CF3CF=CH2). Fig. 5 shows Sensor A's sensitivity to different concentration of R134a. Leak Detector with a typical Sensor A can detect 0.6 g/year leak rate of R134a intermittently for at least 3 months. Fig. 6 shows sensitivity of Sensor A to 100 ppm different gas/vapor. The sensor shows no response to 100 ppm of isobutene (R600a), hydrogen, isopropanol and methane (CH4), but good response to 100 ppm R134a and 100 ppm R1234yf. Sensors with Material B show similar sensor performance as Sensor A, moreover it is possible to operate Sensor B at higher temperature from 800 C to 1200 C to get high sensitivity.

In the foregoing specification, the invention is described with reference to specific embodiments thereof, but those skilled in the art will recognize that the invention is not limited thereto. Different features and aspects of the above-described disclosure may be used individually or jointly.

## Claims

1. Halogenated gas sensor for detecting halogenated gas, comprising at least a first metal electrode and a second metal electrode, which are connected with a sensing material, which comprises at least one of NaAlSiO4, KAlSiO4, RbAlSiO4, CsAlSiO4,
wherein the sensing material comprises at least a first component (A) made from a molecular sieve (3A), which is heated to a first temperature of several hundred °C, maintained at said first temperature for several, and preferably 3 hours, thereafter being heated to a second temperature, which is higher than the first temperature and kept at said second temperature for a second time, which preferably corresponds to said first time,
**characterized in that**
said first component contains NaAlSiO4 and KAlSiO4, preferably at a ratio of 1:1.

2. The sensor of claim 1, wherein the sensing material is in the form of a bead, in which the two electrodes are at least partially embedded.

3. The sensor of claim 1 or 2, wherein one of said two electrodes is a coil surrounding the other of the two electrodes as a center electrode.

4. The sensor of one of claims 1-3, wherein the first electrode is made of or comprises platinum and/or where the second electrode is made of or comprises platinum.

5. The sensor of one of claims 1-4, comprising a voltage source connected to at least the first electrode and configured to heat the electrode by current or voltage applied to said electrode to a temperature in the range of 400°C-1200°C, 400°C-1000°C, 600°C-1200°C or 600°C-1000°C.

6. The sensor of one of claims 1-5, wherein the first sensor is in the form of a coil having a first end and an opposing second end, said two ends being connected to a voltage source (1), said second electrode being a center electrode in the form of a longitudinal straight element or bar extending through the center of the coil (4) along the longitudinal axis of the coil, said coil and center electrode being surrounded by and embedded in said sensing material.

7. Method of detecting halogenated gas with a sensor according to one of claims 1-6, wherein said sensing material is heated to a temperature in the range of 400°C-1000°C, 400°-1200°C, 600°C-1000°C or 600°C-1200°C by applying a current or voltage to said first sensor or coil.

8. The method of detecting halogenated gas according to claim 7, wherein the current through the coil after exposing the sensor to the gas to be detected is divided by the current through the coil before the sensor is exposed to the gas to be detected.

9. Method of manufacturing a halogenated gas sensor according to one of claims 1-6, wherein the sensing material comprises at least a first component (A) made from a molecular sieve (3A), which is heated to a first temperature of several hundred °C, maintained at said first temperature for a first time of several, and preferably 3 hours, thereafter being heated to a second temperature, which is higher than the first temperature and kept at said second temperature for a second time, which preferably corresponds to said first time,
**characterized in that**
said first component contains NaAlSiO4 and KAlSiO4.

10. The method of claim 9, wherein said first component is subsequently ground to fine particles with an average size of below 5 µm, preferably about 3 µm.

11. The method of claim 9 or 10, wherein said first component contains NaAlSiO4 and KAISiO4 at a ratio of 1:1.

12. The method of one of claims 9-11, wherein the sensing material comprises at least a second component (B) which is prepared with an ion exchange performed with a molecular sieve (4A) and CsNO₃, said molecular sieve and CsNO₃ preferably being mixed in deionized water.

13. The method of claim 12, wherein the mixed suspension of molecular sieve, CsNO₃ and deionized water is stirred for several, and preferably 24, hours, whereafter the suspension is preferably centrifuged, thereafter preferably being heated to a first temperature from 400 °C to 1000 °C and preferably about 900°C for at least one hour and preferably for two hours, and thereafter heated to a second temperature higher than said first temperature, preferably to 1100°C, for several additional hours, and preferably for about 3 hours.

14. The method of claim 13, wherein said second component (B) is ground after said heat treatment to fine particles with an average size of a few and preferably about 4 µm.

15. The method of one of claims 9-14, wherein said first component (A) and/or said second component (B) are mixed with a vehicle to a slurry, said vehicle preferably being in the range of 5%-10% weight hydroxypropyl cellulose dissolved in water, the weight ratio of the mixture of said components (A) and/or (B) to said vehicle being about 2:1.

## Patentansprüche

1. Sensor für halogeniertes Gas zum Detektieren von halogeniertem Gas, das mindestens eine erste Metallelektrode und eine zweite Metallelektrode aufweist, die mit einem Sensiermaterial verbunden sind, das mindestens eines von NaAlSiO4, KAlSiO4, RbAlSiO4, CsAlSiO4 umfasst,
wobei das Sensiermaterial mindestens eine erste Komponente (A) aus einem Molekularsieb (3A) aufweist, das auf eine erste Temperatur von mehreren hundert °C erhitzt wird, auf dieser ersten Temperatur mehrere und vorzugsweise 3 Stunden gehalten wird, danach auf eine zweite Temperatur erhitzt wird, die höher ist als die erste Temperatur, und auf dieser zweiten Temperatur eine zweite Zeit lang gehalten wird, die vorzugsweise der ersten Zeit entspricht,
**dadurch gekennzeichnet, dass**
die erste Komponente NaAlSiO4 und KAlSiO4 enthält, vorzugsweise in einem Verhältnis von 1:1.

2. Sensor nach Anspruch 1, wobei das Sensiermaterial die Form einer Perle hat, in welche die beiden Elektroden zumindest teilweise eingebettet sind.

3. Sensor nach Anspruch 1 oder 2, wobei eine der beiden Elektroden eine Spule ist, welche die andere der beiden Elektroden als Mittelelektrode umgibt.

4. Sensor nach einem der Ansprüche 1-3, wobei die erste Elektrode aus Platin hergestellt ist oder Platin enthält und/oder die zweite Elektrode aus Platin hergestellt ist oder Platin enthält.

5. Sensor nach einem der Ansprüche 1-4, der eine Spannungsquelle aufweist, die mit mindestens der ersten Elektrode verbunden und so ausgebildet ist, dass sie die Elektrode durch Strom oder Spannung, die an die Elektrode angelegt wird, auf eine Temperatur im Bereich von 400°C-1200°C, 400°C-1000°C, 600°C-1200°C oder 600°C-1000°C erhitzt.

6. Sensor nach einem der Ansprüche 1-5, wobei der erste Sensor die Form einer Spule mit einem ersten Ende und einem entgegengesetzten zweiten Ende hat, wobei die beiden Enden mit einer Spannungsquelle (1) verbunden sind, wobei die zweite Elektrode eine Mittelelektrode in Form eines länglichen geraden Elements oder Stabes ist, der sich durch die Mitte der Spule (4) entlang der Längsachse der Spule erstreckt, wobei die Spule und die Mittelelektrode von dem Sensiermaterial umgeben und darin eingebettet sind.

7. Verfahren zum Detektieren von halogeniertem Gas mit einem Sensor nach einem der Ansprüche 1-6, wobei das Sensiermaterial durch Anlegen eines Stroms oder einer Spannung an den ersten Sensor oder die erste Spule auf eine Temperatur im Bereich von 400°C-1000°C, 400°-1200°C, 600°C-1000°C oder 600°C-1200°C erhitzt wird.

8. Verfahren zum Detektieren von halogeniertem Gas nach Anspruch 7, wobei der Strom durch die Spule, nachdem der Sensor dem zu detektierenden Gas ausgesetzt wurde, durch den Strom durch die Spule geteilt wird, bevor der Sensor dem zu detektierenden Gas ausgesetzt wird.

9. Verfahren zur Herstellung eines Sensors für halogeniertes Gas nach einem der Ansprüche 1-6, wobei das Sensiermaterial mindestens eine erste Komponente (A) aus einem Molekularsieb (3A) aufweist, das auf eine erste Temperatur von mehreren hundert °C erhitzt wird, auf dieser ersten Temperatur für eine erste Zeit von mehreren und vorzugsweise 3 Stunden gehalten wird, danach auf eine zweite Temperatur erhitzt wird, die höher ist als die erste Temperatur, und auf dieser zweiten Temperatur eine zweite Zeit lang gehalten wird, die vorzugsweise der ersten Zeit entspricht,
**dadurch gekennzeichnet, dass**
die erste Komponente NaAlSiO4 und KAlSiO4 enthält.

10. Verfahren nach Anspruch 9, wobei die erste Komponente anschließend zu feinen Partikeln mit einer durchschnittlichen Größe von unter 5 µm, vorzugsweise etwa 3 µm, gemahlen wird.

11. Verfahren nach Anspruch 9 oder 10, wobei die erste Komponente NaAlSiO4 und KAlSiO4 in einem Verhältnis von 1:1 enthält.

12. Verfahren nach einem der Ansprüche 9-11, wobei das Sensierrmaterial mindestens eine zweite Komponente (B) aufweist, die durch einen Ionenaustausch vorbereitet wird, der mit einem Molekularsieb (4A) und CsNO₃ durchgeführt wird, wobei das Molekularsieb und CsNO₃ vorzugsweise in entionisiertem Wasser gemischt werden.

13. Verfahren nach Anspruch 12, wobei die gemischte Suspension aus Molekularsieb, CsNO₃ und entionisiertem Wasser mehrere und vorzugsweise 24 Stunden lang gerührt wird, wonach die Suspension vorzugsweise zentrifugiert wird, danach vorzugsweise mindestens eine Stunde lang und vorzugsweise zwei Stunden lang auf eine erste Temperatur von 400 °C bis 1000 °C und vorzugsweise etwa 900 °C erhitzt wird und danach mehrere weitere Stunden lang und vorzugsweise etwa 3 Stunden lang auf eine zweite Temperatur erhitzt wird, die höher ist als die erste Temperatur, vorzugsweise auf 1100 °C.

14. Verfahren nach Anspruch 13, wobei die zweite Komponente (B) nach der Wärmebehandlung zu feinen Partikeln mit einer durchschnittlichen Größe von einigen wenigen und vorzugsweise etwa 4 µm gemahlen wird.

15. Verfahren nach einem der Ansprüche 9-14, wobei die erste Komponente (A) und/oder die zweite Komponente (B) mit einem Vehikel zu einer Aufschlämmung gemischt werden, wobei das Vehikel vorzugsweise im Bereich von 5 bis 10 Gew.-% in Wasser gelöster Hydroxypropylcellulose liegt, wobei das Gewichtsverhältnis der Mischung der Komponenten (A) und/oder (B) zu dem Vehikel etwa 2:1 beträgt.

## Revendications

1. Capteur de gaz halogéné permettant de détecter du gaz halogéné, comprenant au moins une première électrode métallique et une seconde électrode métallique, qui sont connectées à un matériau de détection, qui comprend au moins un composant parmi NaAlSiO4, KAlSiO4, RbAlSiO4, CsAlSiO4,
dans lequel le matériau de détection comprend au moins un premier composant (A) fabriqué à partir d'un tamis moléculaire (3A), qui est chauffé à une première température de plusieurs centaines de °C, maintenu à ladite première température pendant plusieurs heures, et de préférence 3 heures, puis chauffé à une seconde température qui est supérieure à la première température et maintenu à ladite seconde température pendant une seconde durée qui correspond de préférence à ladite première durée,
**caractérisé en ce que**
ledit premier composant contient du NaAlSiO4 et du KAlSiO4, de préférence selon un rapport de 1:1.

2. Capteur de la revendication 1, dans lequel le matériau de détection se présente sous la forme d'une bille dans laquelle les deux électrodes sont au moins partiellement intégrées.

3. Capteur de la revendication 1 ou 2, dans lequel une certaine desdites deux électrodes est une bobine entourant l'autre des deux électrodes en tant qu'électrode centrale.

4. Capteur de l'une des revendications 1 à 3, dans lequel la première électrode est constituée de ou comprend du platine et/ou dans lequel la seconde électrode est constituée de ou comprend du platine.

5. Capteur de l'une des revendications 1 à 4, comprenant une source de tension connectée à au moins la première électrode et configurée pour chauffer l'électrode, par l'intermédiaire d'une intensité ou d'une tension appliquée à ladite électrode à une température dans la plage de 400 °C à 1 200 °C, de 400 °C à 1 000 °C, de 600 °C à 1 200 °C, ou de 600 °C à 1 000 °C.

6. Capteur de l'une des revendications 1 à 5, dans lequel le premier capteur se présente sous la forme d'une bobine présentant une première extrémité et une seconde extrémité opposée, lesdites deux extrémités étant connectées à une source de tension (1), ladite seconde électrode étant une électrode centrale sous la forme d'un élément rectiligne longitudinal ou d'une barre s'étendant à travers le centre de la bobine (4) le long de l'axe longitudinal de la bobine, ladite bobine et ladite électrode centrale étant entourées par et intégrées dans ledit matériau de détection.

7. Procédé de détection de gaz halogéné à l'aide d'un capteur selon l'une des revendications 1 à 6, dans lequel ledit matériau de détection est chauffé à une température dans la plage de 400 °C à 1 000 °C, de 400 °C à 1 200 °C, de 600 °C à 1 000 °C ou de 600 °C à 1 200°C en appliquant une intensité ou une tension audit premier capteur ou à ladite bobine.

8. Procédé de détection de gaz halogéné selon la revendication 7, dans lequel l'intensité traversant la bobine après exposition du capteur au gaz à détecter est divisée par l'intensité traversant la bobine avant que le capteur ne soit exposé au gaz à détecter.

9. Procédé de fabrication d'un capteur de gaz halogéné selon l'une des revendications 1 à 6, dans lequel le matériau de détection comprend au moins un premier composant (A) fabriqué à partir d'un tamis moléculaire (3A), qui est chauffé à une première température de plusieurs centaines de °C, maintenu à ladite première température pendant une première durée de plusieurs heures, et de préférence de 3 heures, puis chauffé à une seconde température qui est supérieure à la première température et maintenu à ladite seconde température pendant une seconde durée qui correspond de préférence à ladite première durée,
**caractérisé en ce que**
ledit premier composant contient du NaAlSiO4 et du KAlSiO4.

10. Procédé de la revendication 9, dans lequel ledit premier composant est ensuite broyé en fines particules présentant une taille moyenne inférieure à 5 µm, de préférence d'environ 3 µm.

11. Procédé de la revendication 9 ou 10, dans lequel ledit premier composant contient du NaAlSiO4 et du KAlSiO4, selon un rapport de 1:1.

12. Procédé de l'une des revendications 9 à 11, dans lequel le matériau de détection comprend au moins un second composant (B) qui est préparé avec un échange d'ions effectué avec un tamis moléculaire (4A) et du CsNO₃, ledit tamis moléculaire et ledit CsNO₃ étant de préférence mélangés dans de l'eau désionisée.

13. Procédé de la revendication 12, dans lequel la suspension mélangée de tamis moléculaire, de CsNO₃ et d'eau désionisée est agitée pendant plusieurs heures, et de préférence 24 heures, après quoi la suspension est de préférence centrifugée, puis est de préférence chauffée à une première température de 400 °C à 1 000 °C, et de préférence d'environ 900 °C, pendant au moins une heure, et de préférence pendant deux heures, et ensuite chauffée à une seconde température supérieure à ladite première température, de préférence à 1 100 °C, pendant plusieurs heures supplémentaires, et de préférence pendant environ 3 heures.

14. Procédé de la revendication 13, dans lequel ledit second composant (B) est broyé après ledit traitement thermique en particules fines présentant une taille moyenne de quelques µm, et de préférence d'environ 4 µm.

15. Procédé de l'une des revendications 9 à 14, dans lequel ledit premier composant (A) et/ou ledit second composant (B) sont mélangés avec un véhicule pour former une boue liquide, ledit véhicule étant de préférence dans la plage de 5 % à 10 % en poids d'hydroxypropylcellulose dissoute dans de l'eau, le rapport pondéral du mélange desdits composants (A) et/ou (B) audit véhicule étant d'environ 2:1.
